# EUROPEAN PATENT APPLICATION

(11) **EP 4 361 240 A2**
(43) Date of publication of application: **01.05.2024**
(21) Application number: 24150408.3
(22) Date of filing: 17.11.2016
(51) Int. Cl.: C12H 1/00

(54) **PREPARATION OF A STABLE BEER**

(30) Priority: 17.11.2015 EP 15194858
(62) Divisional of application: 16798474.9
(71) Applicant: DSM IP Assets B.V., 6411 TE Heerlen (NL)
(72) Inventor: MUTSAERS, Johanna Henrica Gerdina Maria, 6100 AA Echt (NL); HEIJNE, Wilbert Herman Marie, 6100 AA Echt (NL)
(74) Representative: dsm-firmenich IP

(57) **Abstract**

The present invention relates to a process for preparing beer comprising the steps of preparing a mash, separating wort from the mash, boiling of the wort, fermenting the wort, and preparing the beer, wherein a proline-specific endoprotease which has at least 80% identity to the mature polypeptide sequence of SEQ ID NO: 2 and a polyphenoloxidase are contacted with the mash during preparing of the mash and / or with the wort before wort boiling. The invention also relates to a beer which has a H90 value of between 0 and 7, when measured after forcing at 60 degrees Celsius for 6 days and subsequent storage at 0 degrees Celsius for 1 day.

## Description

The present invention relates to a process for the preparation of a beverage which is stable for a prolonged period of time.

### Background

Haze is a well-known phenomenon in the beverage industry. Haze can be present in beer, wine and fruit juice. Haze formation can occur at different stages during a brewing process. In "Enzymes in food processing" edited by T. Nagodawithana and G. Reed, 3rd edition, Academic press Inc., San Diego, Chapter V, p.448-449, it has been proposed that haze in beer is the result of interactions between beer proteins and polyphenolic procyanidins. It is explained that in beer haze is often formed upon chilling of the beer. Beer is fermented, maturated, cold stabilised and finally packaged often under chilled conditions. To achieve clarity, beer is often filtered while cold. In spite of the filtration, beer can often become cloudy after it is packaged and distributed to customers and chilled again for example before serving. Eventually haze can be even formed in beer when it is not or no longer chilled and sediment may develop. Haze formation is undesirable because the cloudiness caused by haze formation resembles cloudiness produced by microbial spoilage, which is undesirable, especially for bright beers.

WO2002/046381 discloses a process for the reduction of haze in a beverage by adding a proline-specific endoprotease to the beverage. WO2002/046381 further discloses that by reducing haze with proline-specific endoprotease, a high content of anti-oxidants, such as polyphenols are still present in the beverage. These antioxidant polyphenols are considered valuable as a health improving ingredient.

WO2014090803 discloses that when a proline-specific endoprotease and a polyphenol oxidase were added to wort after wort boiling and cooling of the wort, a beer was produced that was more stable over a prolonged period of time compared to beer that was prepared when either proline-specific endoprotease or polyphenol oxidase are added to the wort.

A disadvantage of adding the enzymes proline-specific endoprotease and / or a polyphenol oxidase to wort after wort boiling is that enzyme activity may remain in the final beer produced, since not all enzyme activity may be removed after pasteurization of beer.

There is a need for an alternative process for producing a stable beer.

### Summary

The present invention relates to a process for preparing beer comprising the steps of
a) preparing a mash,
b) separating wort from the mash,
c) boiling of the wort,
d) fermenting the wort, and
e) preparing the beer,
wherein a proline-specific endoprotease which has at least 80% identity to the mature polypeptide sequence of SEQ ID NO: 2 and a polyphenoloxidase are contacted with the mash during preparing of the mash and / or with the wort before boiling of the wort.

Advantageously, it was found that also after prolonged storage less haze was formed in beer that was treated with a proline-specific endoprotease as disclosed herein and a polyphenoloxidase as compared to a beer produced with none or one of the enzymes alone, or compared to a beer prepared by process wherein a proline-specific endoprotease having less than 80% identity to a mature sequence of SEQ ID NO: 2, such proline-specific endoprotease derived from *Aspergillus niger,* and a polyphenoloxidase are used.

The present invention also relates to a beer for instance obtainable by a process as disclosed herein, which has a H90 value of between 0 and 7, when measured after forcing at 60 degrees Celsius for 6 days and subsequent storage at 0 degrees Celsius for 1 day. The beer may further have a H25 value of between 0 and 3.5 when measured after forcing at 60 degrees Celsius for 6 days and subsequent storage at 0 degrees Celsius for 1 day.

Also disclosed herein is a packaging comprising a beer as disclosed herein.

The present disclosure also relates to the use of a proline-specific endoprotease which has at least 80% identity to the mature polypeptide sequence of SEQ ID NO: 2 and a polyphenoloxidase to reduce haze in a beer.

Further, the present disclosure relates to a composition comprising a proline-specific endoprotease which has at least 80% identity to the mature polypeptide sequence of SEQ ID NO: 2 and a polyphenoloxidase.

### Definitions

A "peptide" refers to a short chain of amino acid residues linked by a peptide (amide) bonds. The shortest peptide, a dipeptide, consists of 2 amino acids joined by single peptide bond.

The term "polypeptide" refers to a molecule comprising amino acid residues linked by peptide bonds and containing more than five amino acid residues. The term "protein" as used herein is synonymous with the term "polypeptide" and may also refer to two or more polypeptides. Thus, the terms "protein" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity in the presence of a specific substrate under certain conditions may be referred to as enzymes. It will be understood that, as a result of the degeneracy of the genetic code, a multitude of nucleotide sequences encoding a given polypeptide may be produced.

A "polyphenol" is defined as a compound having a chemical structure, comprising at least two aromatic rings substituted with at least one hydroxyl group or having a chemical structure which contains at least one aromatic ring substituted with at least two hydroxyl groups. Examples of polyphenols are tannins and flavonoids, for example catechins, flavonols and anthocyanins.

"Polyphenol oxidase" is a group of enzymes oxidizing mono- or di-phenols or derivatives thereof. A polyphenoloxidase may belong to E.C. enzyme classification E.C 1.10.3.1 (catechol oxidase), EC 1.10.3.2 (laccase) or E.C. 1.14.18.1 (monophenol mono-oxidase). Polyphenol oxidases are widespread in nature and occurring in plants, bacteria and fungi. Polyphenol oxidase in a process as disclosed herein may be derivable from *Trametes sp.* such as *Trametes versicolor, and Trametes villosa, Agaricus bisporus, Streptomyces coelicolor, Pleurotus sp.* such as *P. ostreatus, Pycnoporus, Polyporus, Myceliophthora thermophilia, Aspergillus sp, Neurospora,* and *Bacillus sp.*

A "proline-specific endoprotease" (PEP) is a protease that hydrolyses a protein or peptide at a position where the protein or peptide contains a proline-residue. A proline-specific endoprotease may have proline-specific endopotease and/or proline-specific oligopeptidase activity (EC3.4.21.26). A proline-specific endoprotease is preferably an enzyme that hydrolyses a peptide bond at the carboxy-terminal end of proline residues, resulting in a peptide and/or polypeptide fragment with a C-terminal proline.

A "pure enzyme" is synonymous to "pure polypeptide" and means a polypeptide that is removed from at least one component, e.g. other polypeptide material, with which it is naturally associated. The polypeptide may be free of any other impurities. The polypeptide may be at least 50% pure, e.g., at least 60% pure, at least 70% pure, at least 75% pure, at least 80% pure, at least 85% pure, at least 80% pure, at least 90% pure, or at least 95% pure, 96%, 97%, 98%, 99%, 99.5%, 99.9% as determined by SDS-PAGE or any other analytical method suitable for this purpose and known to the person skilled in the art. An isolated polypeptide may be produced by a recombinant host cell.

The term "substantially pure" with regard to polypeptides refers to a polypeptide preparation which contains at the most 50% by weight of other polypeptide material. The polypeptides disclosed herein are preferably in a substantially pure form. In particular, it is preferred that the polypeptides disclosed herein are in "essentially pure form", i.e. that the polypeptide preparation is essentially free of other polypeptide material. Optionally, the polypeptide may also be essentially free of non-polypeptide material such as nucleic acids, lipids, media components, and the like.

Sequence identity, or sequence homology are used interchangeable herein. For the purpose of this invention, it is defined here that in order to determine the percentage of sequence homology or sequence identity of two amino acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more amino acids. The sequence identity is the percentage of identical matches between the two sequences over the reported aligned region. The percent sequence identity between two amino acid sequences may be determined using the Needleman and Wunsch algorithm for the alignment of two sequences. (Needleman, S. B. and Wunsch, C. D. (1970) J. Mol. Biol. 48, 443-453). Both amino acid sequences can be aligned by the algorithm. The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice,P. Longden,I. and Bleasby,A. Trends in Genetics 16, (6) pp276-277, http://emboss.bioinformatics.nl/). For protein sequences EBLOSUM62 is used for the substitution matrix. The skilled person will appreciate that all these different parameters will yield slightly different results but that the overall percentage identity of two sequences is not significantly altered when using different algorithms.

After alignment by the program NEEDLE as described above the percentage of sequence identity between a query sequence and a sequence of the invention is calculated as follows: Number of corresponding positions in the alignment showing an identical amino acid in both sequences divided by the total length of the alignment after subtraction of the total number of gaps in the alignment. The identity as defined herein can be obtained from NEEDLE by using the NOBRIEF option and is labeled in the output of the program as "longest-identity".
The protein sequences of the present invention can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, word length = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., XBLAST and NBLAST) can be used. See the homepage of the National Center for Biotechnology Information at http://www.ncbi.nlm.nih.gov/.

### Detailed description

The present invention relates to a process for preparing beer comprising the steps of
a) preparing a mash,
b) separating wort from the mash,
c) boiling of the wort,
d) fermenting the wort, and
e) preparing the beer,
wherein a proline-specific endoprotease which has at least 80% identity to the mature polypeptide sequence of SEQ ID NO: 2 and a polyphenoloxidase are contacted with the mash during preparing of the mash and / or with the wort before boiling of the wort.

Surprisingly, it was found that when a proline-specific endoprotease which has at least 80% identity to SEQ ID NO: 2 and a polyphenoloxidase are contacted with the mash during preparing of the mash and / or with the wort before wort boiling a beer could be produced that was stable over a prolonged period of time. Surprisingly, a beer prepared in a process as disclosed herein had lower haze values compared to a beer prepared in a process wherein a proline-specific endoprotease which has less than 80% identity to the mature polypeptide sequence of SEQ ID NO: 2, for instance a proline-specific endoprotease derived from *Aspergillus niger,* and a polyphenol oxidase are present during mashing or in the wort before wort boiling. Haze values as used herein are H90 and / or H25 values measured after forcing at a temperature of 60 degrees Celsius for 6 days, and subsequent storage of the beer at 0 degree Celsius for 1 day

A prolonged storage of beer may be beer that is stored for at least one month, or at least two, three, four, five or at least six months, or a beer that is stored for six to twelve months or a beer stored for twelve to eighteen months. Haze in beer may be measured by determining the H90 and H25 expressed in ECB units as disclosed in Materials and Methods section. A common method for estimating whether a beer is stable for a prolonged period of time is by determining the stability using an accelerated stability or aging method. In an accelerated stability method exemplified herein, the H90 and H25 values in beer are determined after keeping the beer at a temperature of 60 degrees Celsius for 6 days, and subsequent storage of the beer at 0 degree Celsius for 1 day. Keeping beer at a temperature of 60 degrees Celsius for 6 days is also known as forcing beer at a temperature of 60 degrees Celsius for 6 days. Advantageously the H90 values in the beer after the accelerated stability or aging method are between 0 and 7, for instance between 0.5 and 5, for instance between 1 and 4. Advantageously the H25 values of beer after the accelerated stability method are between 0 and 3, for instance between 0.5 and 2.

It was found advantageous to contact the proline-specific endoprotease and the polyphenoloxidase with the mash during the preparation of the mash and / or with the wort before wort boiling, because after boiling of the wort no residual enzyme activity of the enzymes that are present in the mash and / or in the wort remains in the final beer. Residual enzyme activity is measured a proline-specific endoprotease activity in NASU as disclosed in the Materials and Methods section.

Preparing a mash in a process as disclosed herein may comprise preparing a mash from a grist of unmalted cereals, from malted cereals or from a mixture of malted and unmalted cereals. A grist as used herein can also be a malt. Examples of cereals from which a mash and subsequently beer can be prepared are barley and wheat. In addition, mash and subsequently beer can be prepared with adjuncts such as maize, rice, sorghum, oats, or cassava.

Mashing, i.e. the process for preparing a mash, typically involves pauses (rests) at certain temperatures, for instance a pause at 43 to 51°C, a pause at 62 to 65°C, a pause at 72 to 74°C and / or a pause at 77-78°C.

Contacting the proline-specific endoprotease and the polyphenol oxidase as used herein may comprise adding the proline-specific endoprotease and the polyphenol oxidase to the mash or to the wort before wort boiling. Advantageously, the proline-specific endoprotease and the polyphenol oxidase are added to the mash at the start of the step of preparing a mash, for instance at a first pause at 43°C to 51°C.

In one embodiment in a process as disclosed herein contacting the proline-specific endoprotease and the polyphenol oxidase is performed at a temperature of at least 50°C, 60°C or 70°C, for instance a temperature of between 40°C and 85 °C during at least part of said contacting. Contacting the proline-specific endoprotease and the polyphenol oxidase may be performed during any suitable period of time. For instance, contacting the proline-specific endoprotease and the polyphenol oxidase may be performed during the time needed to prepare a mash.

After mashing, the wort is separated from the mash by known methods in the art, for instance by filtration or through a Lautertun or mash filter.

Also disclosed herein is a composition comprising a proline-specific endoprotease which has at least 80% identity to the mature polypeptide sequence of SEQ ID NO: 2 and a polyphenoloxidase. Advantageously a composition as disclosed herein is a mash or a wort.

In one embodiment a process as disclosed herein comprises boiling of the wort. Boiling of the wort denatures any enzyme activities in the wort, for instance proline-specific endoprotease and / or polyphenoloxidase activity as disclosed herein. After boiling of the wort, the wort is cooled before fermenting of the wort. Accordingly, a process for preparing beer comprises a step of cooling the wort. Cooling of the wort typically comprises bringing the wort to a temperature of between 5°C and 20°C, such as between 8°C and 18°C or between 10°C and 15°C. After cooling the wort, a process for preparing beer as disclosed herein comprises fermenting the wort. Fermenting wort is typically started by addition of yeast which converts the available sugars into alcohol, also known as primary fermentation. Fermenting of the wort in a process for preparing beer is usually performed at a temperature of between 5°C and 20°C, for instance between 8°C and 18°C, for instance between 10°C and 14°C. After fermentation a process for preparing beer usually comprises a maturation phase which is also known as the secondary fermentation. During maturation undesirable flavour components such as diketones are usually converted into better tasting components. Following maturation, a process for preparing beer usually may comprise a stabilisation phase. During stabilisation the formation of polyphenol-protein aggregates is promoted enabling precipitation. Optionally, a process for preparing beer comprises a step of filtration, for instance after stabilisation. Usually the beer is packaged after stabilisation and / or filtration for instance in a bottle, a can or a keg. Usually, a process for preparing beer also comprises pasteurizing the beer.

Beer as used herein can have all possible alcohol contents, for instance from 0 to 12 v/v%, such as from 1 to 10 v/v%, or from 2 to 8 v/v%, or from 3 to 6%.

In one embodiment in a process as disclosed herein the proline-specific endoprotease and/or the polyphenol oxidase remain active at a temperature of at least 50°C, 60°C, 70°C, 71°C, 72°C, 73°C, 74°C, 75°C, 76°C, 77°C or at least 78°C.

In one embodiment a proline-specific endoprotease as disclosed herein has at least 60%, 65% 70%, 75%, or at least 80% residual activity when kept in a buffer of 0.1 M NaAc pH 4.5, with 50 mM NaCl at 65°C for 15 minutes. Advantageously the enzyme activity is measured with Acetyl AlaAlaPro-para-nitroaniline (Ac-AAP-pNA) as a substrate in buffer of 0.1 M NaAc pH 4.5, with 50 mM NaCl (NASU).

A proline-specific endoprotease advantageously is a polypeptide having proline-specific endoprotease activity which has least 80%, 85%, 86%, 87%, 88%, 89%, 90%, 91%, 92%, 93%, 94%, 95%, 96%, 97%, 98% or 99% identity to the mature polypeptide sequence of SEQ ID NO: 2. The mature polypeptide sequence of SEQ ID NO: 2 comprises, or has amino acids 36 to 526 of SEQ ID NO: 2, wherein methionine at position 1 in SEQ ID NO: 2 is counted as 1.

A proline-specific endoprotease as disclosed herein may be derived from *Rasamsonia emersonii.* The wording "derived" or "derivable" from with respect to the origin of a polypeptide as disclosed herein, means that when carrying out a BLAST search with a polypeptide according to the present invention, the polypeptide according to the present invention may be derivable from a natural source, such as a microbial cell, of which an endogenous polypeptide shows the highest percentage homology or identity with the polypeptide as disclosed herein.

The proline-specific endoprotease and/or polyphenoloxidase advantageously have a pH optimum for activity at a pH below pH 7. For example, the proline specific endoprotease has a pH optimum between 2 and 7, for instance between 3 and 6. The polyphenoloxidase may have a pH optimum between 4 and 7. The pH optima of proline-specific endoprotease and / or polyphenoloxidase activity are measured according to the procedures outlined in the Materials and Methods section.

The proline-specific protease and the polyphenoloxidase may be contacted in any suitable amount and is, among other factors, dependent on the amount of proteins and polyphenols present in the starting material. A suitable amount of proline-specific protease may be from 500 to 100,000 pNASU/kg, such as from 1000 to 50,000 pNASU/kg grist, such as from 2000 to 40,000 pNASU/kg grist, such as from 3000 to 30,000 pNASU/kg grist. A suitable amount of polyphenoloxidase may be from 1 to 500 mg / kg, such as from 2 to 100 mg / kg grist, such as from 3 to 50 mg / kg grist.

The enzymes proline-specific endoprotease and polyphenoloxidase as disclosed herein can be produced by any fermentation process known in the art. Fermentation conditions typically depend on the host organism wherein the enzymes are produced. The enzymes can be expressed in a suitable host organism such as a *Bacillus, Pichia, Trichoderma sp,* or *Aspergillus* sp., such as *Bacillus subtilis, Pichia pastoris, Penicillium chrysogenum, Trichoderma reseei, Aspergillus niger* or *Aspergillus oryzae.* The enzyme can be recovered from culture broths by well-known methods, such as ammonium sulphate or ethanol precipitation, acid extraction, or anion or cation exchange chromatography. High performance liquid chromatography (HPLC) may be employed for purification.

The enzymes proline-specific endoprotease and polyphenoloxidase used in a process as disclosed herein may be pure or substantially pure enzymes.

A process as disclosed herein comprises reducing haze in beer. In this description, the words haze, turbidity and cloudiness are used interchangeably. Haze, turbidity or cloudiness can be formed as the result of protein-polyphenol or polyphenol-polyphenol interactions.

In one embodiment beer prepared in a process as disclosed herein has a H90 value of between 0 and 7, such as between 1 and 6, such as between 2 and 5, when measured after forcing at 60 degrees Celsius for 6 days and subsequent storage at 0 degrees Celsius for 1 day.

A beer prepared in a process as disclosed herein may further have a H25 value of between 0 and 3.5, such as between 0.5 and 2, when measured after forcing at 60 degrees Celsius for 6 days and subsequent storage at 0 degrees Celsius for 1 day

It was found that beer prepared in a process as disclosed herein was stable during prolonged storage.

The present disclosure also relates to a beer obtainable by a process as disclosed herein. It was advantageously found that a beer of the present invention was stable, i.e. during storage less haze was formed compared to a beverage which was prepared with none of the enzymes proline-specific protease or polyphenoloxidase or either proline-specific protease or polyphenoloxidase alone. In addition less haze was formed in beer prepared in a process as disclosed herein, compared to beer prepared in a process wherein a proline-specific endoprotease having less than 80% identity to a mature sequence of SEQ ID NO: 2, for instance proline-specific endoprotease derived from *Aspergillus niger* (eg. Brewers Clarex^{®}), and a polyphenoloxidase is used. Accordingly, in one aspect the present disclosure relates to a beer, for instance a beer obtainable by a process as disclosed herein, which has a H90 value of between 0 and 7, such as between 1 and 6, or between 2 and 5, or between 3 and 4, when measured after forcing at 60 degrees Celsius for 6 days and subsequent storage at 0 degrees Celsius for 1 day. A beer as disclosed herein further has a H25 value of between 0 and 3.5, for instance between 0.5 and 2 when measured after forcing at 60 degrees Celsius for 6 days and subsequent storage at 0 degrees Celsius for 1 day. In addition no enzyme activity was found in the final beer prepared, wherein the enzyme activity is proline-specific endoprotease activity measured with Acetyl AlaAlaPro-para-nitroaniline (Ac-AAP-pNA) as a substrate in buffer of 0.1 M NaAc pH 4.5, with 50 mM NaCl (NASU).

The beer as disclosed herein, for instance a beer obtainable by process as dislosed herein will typically be packaged. Any suitable packaging may be used, for example a bottle, a keg or a can. Beer as disclosed herein may also be packaged in a bulk-tank. Accordingly, in one aspect the present disclosure provides a packaging comprising the beer as disclosed herein, for example a bottle, a keg or a can comprising such beer as disclosed herein. In one aspect the present disclosure also relates to the use of a proline-specific endoprotease which has at least 80% identity to the mature polypeptide sequence of SEQ ID NO: 2 and a polyphenoloxidase to reduce haze in a beverage. Accordingly, the present disclosure relates to a method for reducing haze in a beverage wherein a proline-specific endoprotease which has at least 80% identity to the mature polypeptide sequence of SEQ ID NO: 2 and a polyphenoloxidase are used. A beverage as used herein may be a beer. The use of a proline-specific endoprotease which has at least 80% identity to the mature polypeptide sequence of SEQ ID NO: 2 and a polyphenoloxidase to reduce haze or a method for reducing haze as disclosed herein may comprise a process and all features of such process as disclosed herein.

### EXAMPLES

### MATERIALS & METHODS

### Enzymes

A commercial sample of proline-specific endoprotease from *Aspergillus niger,* Brewers Clarex^{™} was used. The activity was 16500 pNASU/g.
Proline-specific endoprotease *Rasmansonia emersonii* (PEP-R.e.) was obtained as described below. The activity of the PEP-R.e. enzyme preparation used in Example 3 was 2600 pNASU/g. The activity of the PEP-R.e. enzyme preparation in Example 4 was 30.000 pNASU/g.

Laccase M120 (not less than 108,000 POU / g product) is a polyphenoloxidase obtained from Amano Enzyme (Chipping Norton, UK).

### Proline-specific endoprotease activity (pNASU )

Culture supernatant containing PEP as produced in Example 1.2 and 1.3 is diluted in 0.1 M sodium acetate buffer at pH 4.5 with 50 mM NaCl. 100 µL of diluted PEP was incubated with 100 µL 6 mM Ac-AAP-pNA (acetyl-AlaAlaPro-paranitroaniline from Selleckchem or CPC Scientific; purity >95.0% based on HPLC analysis) in 0.1 M NaAc buffer at pH4.5 with 50 mM NaCl, in a Nunc 96 well flat bottom MTP (micro-titer plate). After 60 minutes at 20 °C the reaction was stopped by adding 40 µL of 1 M HCl. The pNA which had been liberated by PEP was measured in a Tecan MTP spectrophotometer at 405 nm (A405) (www.tecan.com). The blank was prepared by mixing the diluted culture supernatant with the substrate solution which had been mixed with the HCl solution beforehand. The activity is expressed in pNASU's. 1pNASU is the amount of enzyme which liberates from Ac-AAP-pNA in 1 hour the amount of pNA that corresponds to an increase in absorption at 405 nm of 1 OD, using the conditions as described above. The A405 should not be below the blank value at the start of the reaction, or above 2.5 at the end of the reaction, nor may the A405 exceed the linear range of the spectrophotometer that is used.

### Polyphenoloxidase activity (POU)

Laccase activity was measured according to the instruction from Amano (E211: The assay method for laccase activity = P-4AA method) by monitoring the increase of quinone-imine dye absorbance at 505 nm under the assay conditions. 4-amino-antipyrine + phenol + O₂ → quinone-imine dye + H₂O.
One laccase unit (POU) is defined as the amount of the enzyme contained in 1 ml of the reaction mixture, the absorbance of which increases at the rate of 0.1 per minute under the assay conditions.

### Turbidity measurements

The turbidity or haze was measured with a Haffman's VOS Rota 90/25 dual angle turbidity meter. The two angles, 90 and 25 degrees, are indicated as H90 and H25. Calibration of the Haffman's VOS Rota 90/25 dual angle turbidity meter was performed according to Analytica EBC method 9.29 using AEPA-1 standards. The result of the haze measurement (H90 and H25 values) was expressed in EBC units. EBC stands for European Brewing Convention.

### EXAMPLE 1. Cloning, Expression and Recovery of Proline-specific endoprotease from Rasamsonnia emersonii (PEP-R.e.)

### Example 1.1. Cloning and Expression

The protein sequence of proline-specific endoprotease (PEP) *Rasamsonia emersonii* is shown in SEQ ID NO: 2.

A codon-adapted DNA sequence for expression of this protein in *Aspergillus niger* was designed containing additional restriction sites for subcloning in an *Aspergillus* expression vector. Codon adaptation was performed as described in WO 2008/000632. The codon optimized DNA sequence for *A. niger* of the gene encoding the PEP-R.e. protein of SEQ ID: NO: 2 is shown in SEQ ID NO: 1.

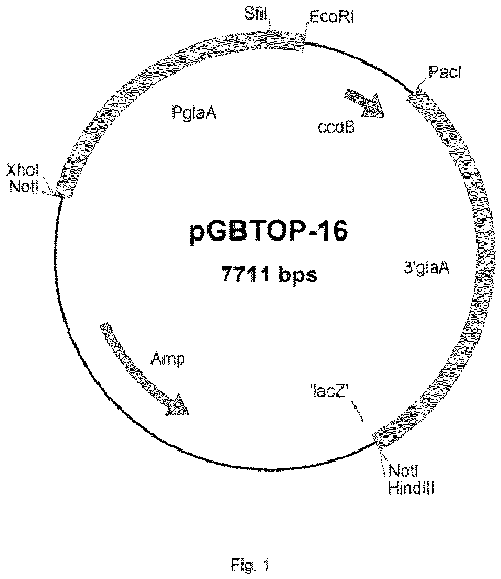

The translational initiation sequence of the glucoamylase glaA promoter was modified into 5'-CACCGTCAAA ATG-3' (SEQ ID NO: 3) and an optimal translational termination sequence 5'-TAAA-3' was used in the generation of the expression construct (as also detailed in WO2006/077258). A DNA fragment containing a.o. part of the glucoamylase promoter and the PEP-R.e. encoding gene was synthesized completely, purified and digested with EcoRI and PacI. The pGBTOP-16 vector (Figure 1) was linearized by EcoRI/Pacl digestion and the linearized vector fragment was subsequently purified by gel-extraction. The DNA fragment containing the PEP-R.e. coding region was cloned into the pGBTOP-16 vector resulting in pGBTOP-PEP. Subsequently, *A. niger* GBA 306 (Δ*gla*A, Δ*pep*A*, ΔhdfA,* adapted BamHl amplicon, ΔamyBll, ΔamyBl, ΔamyA alpha-amylase and glucoamylase negative strain) was transformed with linearized pGBTOP-PEP vector by Notl-digestion, in a co-transformation protocol with linearized pGBAAS-4, with strain and methods as described in WO 2011/009700 and references therein, and selected on acetamide containing media and colony purified according to standard procedures. Transformation and selection was performed as described in WO 98/46772 and WO 99/32617. Strains containing the PEP gene encoding PEP-R.e. were selected via PCR with primers specific for the PEP gene to verify presence of the pGBTOP-PEP expression cassette. A single transformant was selected and further replica-plated to obtain a single strain inoculum.

### Example 1.2. Production of PEP-R.e. in A. niger strain in shake flasks

Fresh *A. niger* spores as prepared in Example 1.1. were used. 4 shake flasks with 100 ml fermentation medium 1 (10 % w/v Corn Steep Solids, 1 % w/v glucose.H₂O, 0.1 % w/v NaH₂PO₄.H₂O, 0.05 % w/v MgSO₄.7H₂O, 0.025 % w/v Basildon, pH 5.8) in 500 ml shake flasks with baffle were inoculated with 10⁷ spores. These pre-cultures were incubated at 34 °C and 170 rpm for 16-24 hours. From the pre-cultures, 50 ml was used for inoculation of 1 shake flasks with 1 liter Fermentation medium 2 (15 % w/v maltose, 6 % w/v bacto-soytone, 1.5 % w/v (NH₄)₂SO₄, 0.1 % w/v NaH₂PO₄.H₂O, 0.1 % w/v MgSO₄.7H₂O, 0.1 % w/v L-arginine, 8 ‰ w/v Tween-80, 2 ‰ w/v Basildon, 2 % w/v MES pH 5.1) in a 5 liter shake flask size and shaken at 34 °C and 170 rpm. After 3, 4, 5, and 6 days incubation the pH of the culture was lowered to pH 5.0 using 2 N HCl and samples from each of these time points were analysed for PEP activity. 50 mL samples were taken and the supernatant was separated from the biomass by centrifugation and subsequent filtering. The sample with the highest activity was used to characterize the PEP produced.

### Example 1.3. Production of PEP-R.e. in A. niger strain in stirred tank reactor

*Aspergillus niger* prepared is Example 1.1. was fermented in a suitable culture medium in a stirred tank reactor, comprising glucose, yeast extract, antifoaming agent, and trace elements. Dissolved oxygen, temperature and the pH were set at suitable values and measured periodically. After fermentation, PEP-R.e. enzyme was recovered from the fermentation broth by filtration. The activity of the enzyme preparation was 2600 pNASU/g. The filtrated enzyme preparation was further concentrated by ultrafiltration and formulated with 50 w/w% glycerol. The activity of the concentrated PEP-R.e. enzyme preparation was 30.000 pNASU/g

### EXAMPLE 2. Thermal stability of proline-specific endoprotease from Rasamsonia emersonii (PEP-R.e.)

To assess the thermal stability of PEP-R.e. the activity assay was preceded by an incubation of 100 µL aliquots of a tenfold dilution of the culture supernatant produced in Example 1.2 in buffer (0.1 M NaAc pH 4.5, with 50 mM NaCl) at 55°C and 65°C for 15 min in a PCR plate in a PCR machine. After the 15 min incubation the samples were rapidly cooled to 25°C in the PCR machine. The pNASU/mL of every sample was measured. The initial activity measured before incubation at elevated temperature (0 minutes) is used as reference (100%) to determine the residual activity.

**Table 1. Proline-specific endoprotease activity of PEP-R.e. after 15 minutes incubation at 55°C and 65°C**

| Description | Residual activity after 15' at 55°C (pNASU) | Residual activity at after 15'at 65°C (pNASU) |
|---|---|---|
| PEP-R.e. | 105% | 84% |

The results in Table1 show that PEP-R.e. derived from *Rasamsonia emersonii* is relatively stable at a temperature of 65 degrees Celsius.

### EXAMPLE 3. Haze formation in beer treated with proline-specific endoprotease, polyphenol oxidase and the combination of these two enzymes

Beer is produced at pilot plant scale. Mashing in at 50°C, pH 5.4, and malt:water ratio of 1:2.2 and 87 kg malt.
The mashing scheme is as follows:
50°C - 15 min
64°C - 30 min
74°C - 15 min
77°C - 5 min

At the start of mashing, proline-specific endoprotease and polyphenol oxidase (PPO) enzymes are added according to the scheme presented in Table 2. Also a mashing without addition of enzymes is performed.
The mash is filtered over a mash filter. The resulting wort is boiled for 1 hour resulting in a density of about 14°P and the bitterness is aimed at 25 ppm by the addition of iso-alpha-acids at the end of boiling.
Fermentation of wort is at 12°C. After fermentation, beer is matured at 0°C for 2 weeks filtered by sheet filtration (Becopad 270 sheet filters).
After filtration and carbonation at 5.6 g/l CO₂, beer is bottled.
The beers are analyzed for initial haze and haze after forcing for 6 days at 60 degree C.
In addition the haze formation on storage in the beer is followed for several months. Furthermore, polyphenols are analyzed.

**Table 2. Scheme of addition of the Brewers Clarex^{™}, PEP-R.e. and PPO at the start of different mashing trials for preparing beer.**

| Trial # | Brewers Clarex^{™} | PEP-R.e. | PPO |
|---|---|---|---|
| 1 | - | - | - |
| 2 | - | - | + |
| 3 | + | - | - |
| 4 | + | - | + |
| 5 | - | + | - |
| 6 | - | + | + |

The PEP-R.e. used herein was prepared as described in Example 1.3. PEP-R.e. and Brewers Clarex^{™} are dosed at 30,000 pNASU/kg of malt Polyphenol oxidase is dosed at 32 mg/kg of malt.

### EXAMPLE 4. Haze formation in beer treated with proline-specific endoprotease, polyphenol oxidase and the combination of these two enzymes

In a similar way as in Example 3, beer was produced in a pilot brewery at 20 HI scale. Mashing in at 50°C, pH 5.4, and malt:water ratio of 1:3 and 350 kg malt.
The mashing scheme was as follows:
50°C - 15 min
64°C - 15 min
74°C - 25 min
78°C - 0 min

At the start of mashing, proline-specific endoprotease and polyphenol oxidase (PPO) enzymes were added according to the scheme presented in Table 3. Also a mashing without addition of these enzymes was performed.
The PEP-R.e. used herein was concentrated and formulated with 50 w/w% glycerol prepared as described in Example 1.3.
PEP-R.e. and Brewers Clarex^{™} were dosed at 30,000 pNASU/kg of malt.
Polyphenol oxidase was dosed at 32 mg/kg of malt.
After wort separation the resulting wort was boiled resulting in a density of about 12°P. Columbus hop at 13,7% alpha acid was added at the onset of boiling.
Fermentation of wort was at 12°C. After fermentation, beer was matured at -1°C for 5 days and filtered.
After filtration and carbonation at 5.2 g/l CO₂, beer was bottled. The bottles were pasteurized applying 15 PU.
The beers were analyzed for initial haze and haze after forcing at 60 degrees Celsius for 6 days and subsequent storing at 0 degree Celsius for 1 day.
The data in Table 3 show that beer prepared after addition of PEP-R.e. and polyphenoloxidase to the mash had the lowest haze formation (H90 and H25 values). Beer prepared in a process wherein PEP-R.e. and polyphenoloxidase are added during mashing resulted in a two to three times lower H90 value and about 8 times lower H25 value compared to beer prepared with Brewers Clarex^{®} and polyphenoloxidase.

**Table 3. Haze formation (H90 and H25 values in EBC unit) in beer prepared after addition of Brewers Clarex^{™}, PEP-R.e. and / or PPO during mashing. H90 and H25 values were measured after forcing for 6 days at 60 degree C and subsequent storage at 0 degree C for 1 day**

| **Trial #** | **Brewers Clarex^{™}** | **PEP-R.e.** | **PPO** | **H90 (EBC unit*)** | **H25 (EBC unit*)** |
|---|---|---|---|---|---|
| 1 | - | - | - | 16.9 | 24.7 |
| 2 | - | - | + | 13.2 | 18.3 |
| 3 | + | - | - | 13.1 | 13.1 |
| 4 | + | - | + | 9.6 | 8.8 |
| 5 | - | + | - | 7.1 | 3.7 |
| 6 | - | + | + | 3.6 | 1.0 |

| | | | | | |
|---|---|---|---|---|---|
| *EBC unit: turbidity unit recommended by the European Brewery Convention (EBC) | | | | | |

## Claims

1. A process for preparing beer comprising the steps of
a. preparing a mash,
b. separating a wort from the mash,
c. boiling of the wort,
d. fermenting the wort, and
e. preparing the beer,
wherein a proline-specific endoprotease which has at least 80% identity to the mature polypeptide sequence of SEQ ID NO: 2 and a polyphenoloxidase are contacted with the mash during preparing of the mash and / or with the wort before boiling of the wort.

2. A process according to claim 1, wherein said contacting is performed at a temperature of at least 50°C, 60°C or 70°C during at least part of said contacting.

3. A process according to claims 1 to 2, wherein the proline-specific endoprotease has at least 60%, 65% 70%, 75%, or at least 80% residual activity when kept in a buffer of 0.1 M NaAc pH 4.5, with 50 mM NaCl at 65°C for 15 minutes.

4. A process according to any one of the claims 1 to 3, wherein the beer comprises less haze compared to a beer prepared by a process wherein a proline-specific endoprotease having less than 80% identity to a mature sequence of SEQ ID NO: 2 and a polyphenoloxidase are contacted with the mash during preparing of the mash and / or with the wort before boiling of the wort.

5. A process according to any one of the claims 1 to 4, wherein the beer has a H90 value of between 0 and 7, when measured after forcing at 60 degrees Celsius for 6 days and subsequent storage at 0 degrees Celsius for 1 day

6. A process according to claim 5, wherein the beer further has a H25 value of between 0 and 3.5 when measured after forcing at 60 degrees Celsius for 6 days and subsequent storage at 0 degrees Celsius for 1 day

7. A beer, optionally obtainable by a process according to any one of the claims 1 to 6, which has a H90 value of between 0 and 7, when measured after forcing at 60 degrees Celsius for 6 days and subsequent storage at 0 degrees Celsius for 1 day.

8. A beer according to claim 7, which further has a H25 value of between 0 and 3.5 when measured after forcing at 60 degrees Celsius for 6 days and subsequent storage at 0 degrees Celsius for 1 day.

9. A packaging comprising a beer according to claim 7 or 8.

10. Use of a proline-specific endoprotease which has at least 80% identity to the mature polypeptide sequence of SEQ ID NO: 2 and a polyphenoloxidase to reduce haze in a beverage.

11. A composition comprising a proline-specific endoprotease which has at least 80% identity to the mature polypeptide sequence of SEQ ID NO: 2 and a polyphenoloxidase.

12. A composition according to claim 11, wherein the composition is a mash or a wort.
